# EUROPEAN PATENT APPLICATION

(11) **EP 3 103 883 A1**
(43) Date of publication of application: **14.12.2016**
(21) Application number: 15196213.1
(22) Date of filing: 08.11.2010
(51) Int. Cl.: C12Q 1/68, C12N 15/10

(54) **STABILIZATION OF RNA IN AND EXTRACTING FROM INTACT CELLS WITHIN A BLOOD SAMPLE**

(30) Priority: 09.11.2009 US 259363 P
(62) Divisional of application: 10782473.2
(71) Applicant: Streck, Inc., Lavista, NE 68128 (US)
(72) Inventor: RYAN, Wayne L, Omaha, NE 68130 (US); FERNANDO, Rohan, Omaha, NE 68136 (US)
(74) Representative: Bawden, Peter Charles

(57) **Abstract**

A method for preserving and processing nucleic acids located within a blood sample is disclosed, wherein a blood sample containing nucleic acids is treated to reduce both blood cell lysis and nuclease activity within the blood sample. The treatment of the sample aids in increasing the integrity and amount of cellular nucleic acids that can be identified and tested while avoiding contamination of the isolated nucleic acids with cell -free nucleic acids. The preferred compositions for preserving and processing contain diazolidinyl urea (DU), EDTA, aurintricarboxylic acid (ATA), glyceraldehyde and sodium floride.

## Description

### CLAIM OF PRIORITY

This application claims the benefit of the filing date of U.S. Provisional Application Serial No. 61/259,363, filed November 9, 2009, the entirety of the contents of this application being hereby expressly incorporated by reference.

### FIELD OF THE INVENTION

This invention relates to the identification and isolation of nucleic acids in blood samples and more particularly to the stabilization of cellular RNA within a blood sample.

### BACKGROUND OF THE INVENTION

Messenger RNA (mRNA) in a cell is a snapshot of the real time activity of its genome, depicting what genes are expressed and to what extent. Profiling of cellular mRNA expression patterns is typically done by use of microarrays, quantitative reverse transcriptase real time PCR and molecular beacons. Profiling of cellular mRNA is becoming important in disease diagnosis, prognosis and in clinical trials for biomarker discovery. Such cellular mRNA profiling has relied on tumor and other biopsy material from affected and unaffected tissues. However, such tissue biopsies may not be readily available and sampling often requires highly invasive procedures of the human body. Therefore, human peripheral blood and blood cells have been explored as a possible source of material for gene expression profiling, which are readily available via a relatively noninvasive procedure. Some issues inherent to gene profiling in blood cells have the significant potential to influence data interpretation. One such issue is related to the handling of blood samples *ex vivo* prior to the extraction of mRNA. Expression levels for many genes in blood cells can be adversely effected by *ex vivo* incubation because of the metabolic stress brought on by the lack of oxygen and glucose sources. The aftereffect of phlebotomy causes the simultaneous degradation of mRNA molecules and the unintended up-regulation of certain genes. Another issue is related to the widely used method for obtaining total RNA from blood cells, which includes density-gradient centrifugation to isolate white blood cells. This method needs equipment beyond what is available in the typical clinical setting and may require shipment to another site for the necessary processing. This causes delays in sample processing and may create significant changes in gene expression profiles. The above observations emphasize the importance of developing blood collection devices capable of stabilizing mRNA expression immediately upon blood draw. By inhibiting cellular metabolism and nuclease (RNase) action, RNA degradation and changes in the mRNA expression profile can be effectively overcome post-phlebotomy.

Several newer technologies have been introduced that have aimed to stabilize whole blood RNA post-phlebotomy. These devices are capable of inhibiting RNase activity in blood cells and cell metabolism by lysing all blood cells at the point of collection and thereby stabilizing the RNA expression profile. However, there are some inherent disadvantages in these blood collection devices. Since all blood cells are lysed at the point of collection, there is a significant introduction of α- and β-globin mRNA, that is released from reticulocytes, which interferes with microarray and real time PCR detection methodologies. Excessive globin mRNA from whole blood decreases mRNA transcript detection sensitivity and increases signal variation on microarrays. Another significant disadvantage of these devices is the inability to utilize molecular beacon technology, where it is imperative to have intact cells so that one can visualize the gene-specific fluorescence staining by histology or by flow cytometry. To circumvent these problems, additional methods are necessary to reduce globin mRNA from whole blood RNA samples obtained using those blood collection devices. As a result, additional costs are incurred and there is increased time required for sample preparation.

A number of patent documents address processes for the stabilization and identification of nucleic acids and other cellular materials and their diagnostic applications. See, generally, U.S. Patent Nos. 5,459,253; 6,043,032; 6,168,922: 6,218,531; 6,602,718; 6,645,731; 6,821,789; 7,282,371; 7,332,288; 7,445,901 and U.S. Patent Publication Nos. 2006/0105372; 2006/0194192; 2008/0119645; and 2008/0318801 all incorporated by reference herein. Notwithstanding the above, there remains a critical need for the development of a blood collection device that stabilizes mRNA expression profiles immediately after a blood draw by completely inhibiting cell metabolism and stabilizing nucleated blood cells while allowing blood cells to remain intact. Such a device would permit isolation of white blood cells by widely used methodologies without compromising the original gene expression profile. The device would further provide stabilized intact cells for use in gene expression profiling using molecular beacon technology.

The use of formaldehyde-donor preservatives for cell and tissue stabilization has been described in U.S. Patent Nos. 5,196,182; 5,260,048; 5,459,073; 6,811,099, 5,849,517; and 6,337,189, incorporated by reference herein. While the use of formaldehyde-donor preservatives for the fixation of cells and tissues is known, formaldehyde-donors have been shown to be less effective in completely inhibiting cell metabolism at least during the first 24 hours of post phlebotomy. Further, the use of formaldehyde-donor preservatives alone have not shown to stabilize mRNA expression patterns in cells within a blood sample post phlebotomy.

The present invention addresses the need for an efficient and consistent method of preserving a blood sample containing diagnostically useful RNA so that the RNA can be effectively isolated and tested, which unexpectedly and surprisingly results in one or any combination of the following: short term inhibition of metabolism (i.e., RNA synthesis); fixation of the cellular RNA within the blood cells to freeze the mRNA expression pattern of the blood cells; stabilization of the RNA that is in the blood cells from nucleases and proteases; prevention of interference from globin RNA and cell-free RNA; and fixation of blood cells to prevent the loss of cellular RNA leaked from white blood cells during transportation or storage of blood specimens.

### SUMMARY OF THE INVENTION

The present invention provides a unique approach to the preservation, isolation, and analysis of nucleic acids. One aspect of the invention involves use of a unique protective agent composition, which includes at least one preservative agent which may include a formaldehyde donor. The nucleic acid may be DNA, RNA, or any combination thereof. The samples from which the nucleic acids may be isolated include any blood sample. The nucleic acids may be cellular nucleic acids (e.g., nucleic acids that are located within cells in vivo as opposed to cell-free nucleic acids found outside of cells in vivo). The method disclosed herein allows for efficient preservation and isolation of cellular nucleic acids while avoiding contamination with undesirable globin mRNA and cell-free nucleic acids that originate at extra-cellular locations in vivo (as compared to cellular RNA that becomes cell-free RNA due to cell metabolism and cell lysis post-blood draw).

In a first aspect, the present invention contemplates a screening method for the identification of a disease state. The screening method includes the step of contacting a drawn blood sample that includes a plurality of blood cells with a protective agent in an amount and for a time sufficient so that RNA synthesis is inhibited for at least two hours. The contact time with the protective agent and amount of protective agent used may also be sufficient so that the blood cells of the drawn blood sample are fixed to substantially prevent contamination of the cellular RNA with cell-free RNA or globin mRNA. Further, any cellular RNA that is within the blood cells at the time of the blood draw may be substantially preserved to freeze the mRNA expression pattern of the blood cells substantially as of the time of the blood draw (e.g., no longer than 10 minutes post-blood draw or even no longer than 5 minutes post-blood draw). The screening method may also include the step of isolating white blood cells from the whole blood by lysing the red blood cells and isolating the white blood cells. The isolated white blood cells may then be treated to extract cellular RNA from the isolated white blood cells.

The protective agent discussed above may include a preservative agent selected from the group consisting of: diazolidinyl urea, imidazolidinyl urea, dimethoylol-5,5dimethylhydantoin, dimethylol urea, 2-bromo-2.-nitropropane-1,3-diol, oxazolidines, sodium hydroxymethyl glycinate, 5-hydroxymethoxymethyl-1-1aza-3, 7-dioxabicyclo[3.3.0]octane, 5-hydroxymethyl-1-1aza-3, 7dioxabicyclo[3.3.0]octane, 5-hydroxypoly[methyleneoxy]methyl-1-1 aza-3, 7dioxabicyclo[3.3.0]octane, quaternary adamantine and any combination thereof. The protective agent may also include one or more metabolic inhibitors selected from the group consisting of: dihydroxyacetone phosphate, glyceraldehyde 3-phosphate, 1,3-bisphosphoglycerate, 3-phosphoglycerate, 2- phosphoglycerate, phosphoenolpyruvate, pyruvate and glycerate dihydroxyacetate, sodium fluoride, K₂C₂O₄ and any combination thereof. The protective agent may further include an nuclease inhibitor selected from the group consisting of: dithiothreitol (DTT), iodoacetamide, iodoacetic acid, heparin, chitosan, cobalt chloride, diethyl pyrocarbonate, ethanol, aurintricarboxylic acid (ATA), glyceraldehydes, sodium fluoride, ethylenediamine tetraacetic acid (EDTA), formamide, vanadyl-ribonucleoside complexes, macaloid, hydroxylamine-oxygen-cupric ion, bentonite, ammonium sulfate, beta-mercaptoethanol, cysteine, dithioerythritol, tris (2-carboxyethyl) phosphene hydrochloride, a divalent cation such as Mg⁺², Mn⁺², Zn⁺², Fe⁺², Ca⁺², Cu⁺² and any combination thereof.

The protective agent may also include an amino acid selected from the group consisting of: isoleucine, leucine, lysine, valine, tryptophan, threonine, phenylalanine, methionine, alanine, histidine, asparagine, aspartate, cysteine, glutamate, glutamine, glycine, proline, serine, tyrosine, arginine, and any combination thereof. The protective agent may include a substance to increase the permeability of cell membranes such as glycerol, dimethyl sulfoxide, chloroquine, BC-30 Tx and any combination thereof. The protective agent may also include a metal ion chelator selected from the group consisting of: ethylene glycol tetraacetic acid (EGTA), 1,2-bis-(o-Aminophenoxy)-ethane-N,N,-N',N'-tetraacetic acid tetraacetoxy-Methyl ester (BAPTA-AM), dietyldithiocarbamate (DEDTC), ethylenediamine tetraacetic acid (EDTA), and any combination thereof. The protective agent may also include an oxidative stress neutralizer selected from the group consisting of: N-acetyl-L-cysteine, D-Mannitol and any combination thereof. The protective agent may also include glycine. The protective agent may also include a protease inhibitor selected from the group consisting of: antipain, aprotinin, chymostatin, elastatinal, phenylmethylsulfonyl fluoride (PMSF), APMSF, TLCK, TPCK, leupeptin, soybean trypsin inhibitor, indoleacetic acid (IAA), E-64, pepstatin, VdLPFFVdL, EDTA, 1,10-phenanthroline, phosphoramodon, amastatin, bestatin, diprotin A, diprotin B, alpha-2-macroglobulin, lima bean trypsin inhibitor, pancreatic protease inhibitor, egg white ovostatin egg white cystatin, and any combination thereof. The protective agent may also include a phosphatase inhibitor selected from the group consisting of: calyculin A, nodularin, NIPP-1, microcystin LR, tautomycin, okadaic acid, cantharidin, microcystin LR, okadaic acid, fostriecin, tautomycin, cantharidin, endothall, nodularin, cyclosporin A, FK 506/immunophilin complexes, cypermethrin, deltamethrin, fenvalerate, bpV(phen), dephostatin, mpV(pic) DMHV, sodium orthovanadate and any combination thereof.

As discussed above, the screening method may include the steps of isolating white blood cells, extracting RNA from the isolated white blood cells and analyzing the extracted RNA. The isolating step, the analyzing step, or both may occur at least 2 hours after the blood sample is drawn. Either or both of the isolating and analyzing steps may occur without freezing the blood sample (e.g. to a temperature colder than about -30°C (more preferably colder than about -70°C)). Either or both of the isolating and analyzing steps may occur at least 3 days after the blood sample is drawn.

The initial contacting step may take place in a blood collection tube into which the blood sample is drawn. The contacting step may take place as the blood sample is drawn. The contacting step may be sufficient so that after a period of at least 3 days from the time the blood sample is drawn, the amount of RNA present in the blood sample is at least about 90% of the amount of RNA present in the blood sample at the time the blood sample is drawn. The contacting step may be sufficient so that after a period of at least 3 days from the time the blood sample is drawn, the amount of RNA present in the sample is about 100% of the amount of RNA present in the sample at the time the blood sample is drawn. The contacting step may be sufficient so that after a period of at least about 3 days from the time the blood sample is drawn, the concentration of RNA relative to the total nucleic acid in the blood sample that is present is at least 10 times the amount of RNA that would be present in the absence of the contacting step. The contacting step may be sufficient so that after a period of at least about 3 days from the time the blood sample is drawn, the concentration of RNA relative to the total nucleic acid in the blood sample that is present is at least about 20 to 50 times the amount of RNA that would be present in the absence of the contacting step.

The preservative agent may be added to a blood collection tube prior to blood draw and one or more additional components may be added to the blood collection tube post-blood draw. All components of the protective agent may be added to a blood collection tube post-blood draw. The preservative agent and one or more nuclease inhibitors may be placed within a blood collection tube in substantially solid form prior to blood draw. All components of the protective agent may be placed within a blood collection tube in substantially solid form prior to blood draw. The protective agent may be made up of multiple components that can be added to a blood collection tube separately or simultaneously prior to blood draw or post-blood draw so that the cellular RNA within the blood cells of a drawn blood sample remains intact.

The screening method step of isolating the white blood cells from a drawn blood sample may include the steps of lysing the red blood cells, lysing the white blood cells, or both. The screening method may further include a step of analyzing (e.g., by quantity, quality, or both) the extracted RNA for the presence, absence or severity of a disease state.

The screening method of the present invention provides a process for preserving a blood sample containing diagnostically useful RNA so that the RNA can be effectively isolated and tested. The preservation technique results in short term inhibition of metabolism (i.e., RNA synthesis), fixation of the cellular RNA within the blood cells to freeze the mRNA expression pattern of the blood cells, protection of the RNA that is in the blood cells from nucleases and proteases, prevention of unwanted interference from globin RNA and cell-free RNA, and fixation of blood cells to prevent the loss of cellular RNA leaked from blood cells during transportation or storage of blood specimens.

### BRIEF DESCRIPTION OF THE DRAWINGS

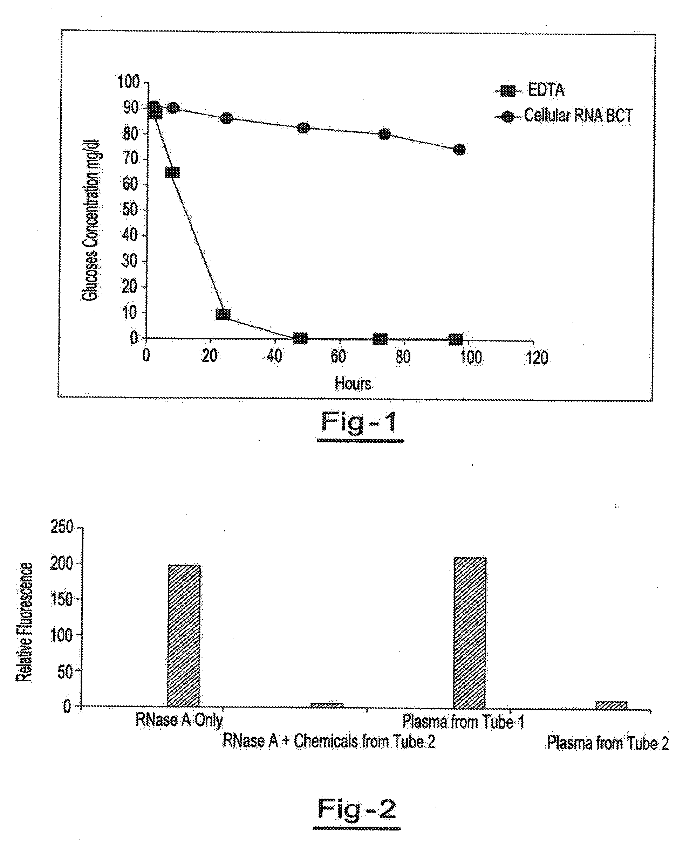

Fig. 1 is a graphic representation showing inhibition of metabolism in blood cells using glucose concentration as an indicator of metabolism.
Fig. 2 is a graphic representation showing the effectiveness of nuclease inhibitors present in the blood collection device in inhibiting RNase activity present in a plasma sample.

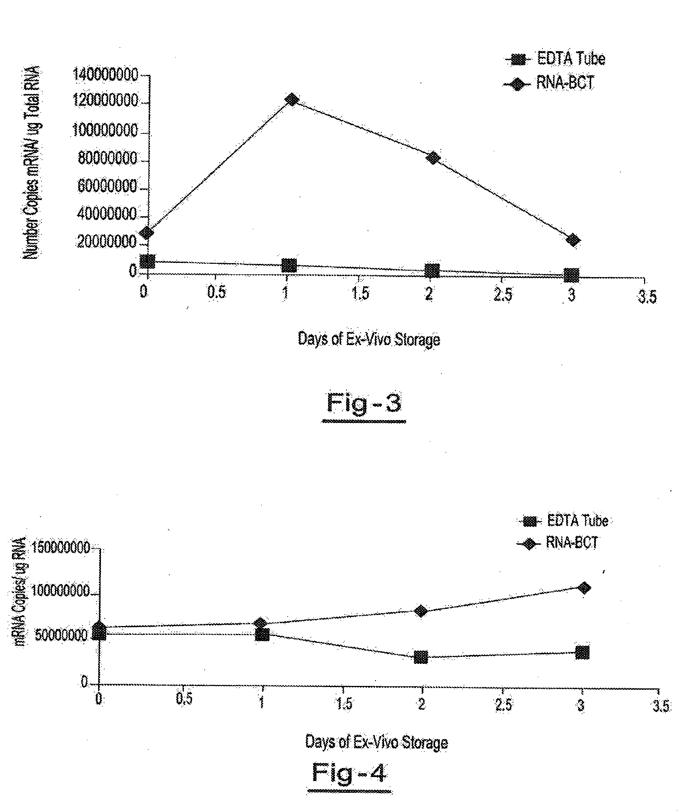

Fig. 3 is a graphic representation showing inhibition of unintended up-regulation of c-fos mRNA in a blood sample collected in accordance with the present invention as compared to collection in a standard EDTA blood collection device. Real Time Reverse Transcriptase PCR technology was used to detect the copy number of c-fos mRNA.
Fig. 4 is a graphic representation showing inhibition of unintended up-regulation of mRNA for glyceraldehydes-3-phosphate dehydrogenase (GADPH) in a blood sample collected in accordance with the present invention as compared to collection in a standard EDTA blood collection device. Real Time Reverse Transcriptase PCR technology was used to detect the copy number of GAPDH mRNA.

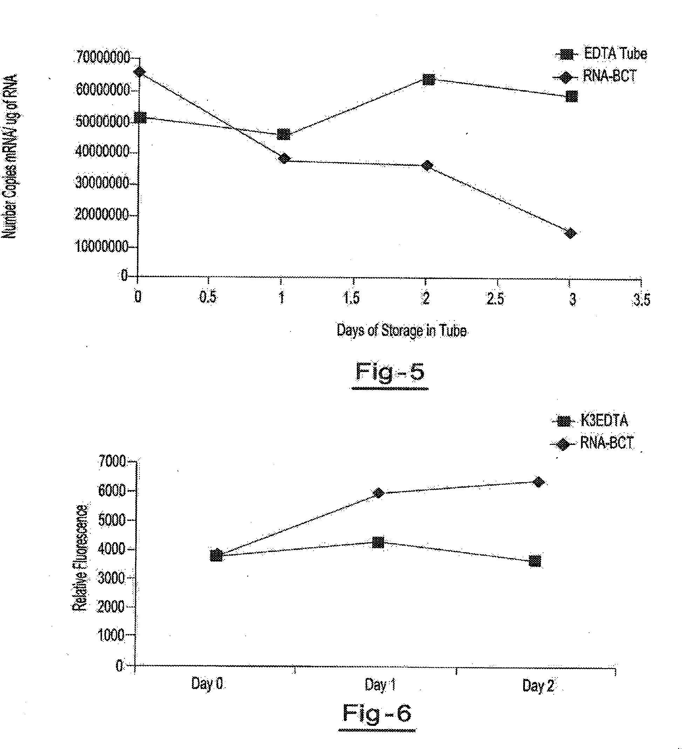

Fig. 5 is a graphic representation showing inhibition of unintended down-regulation of mRNA for RASSF1A in a blood sample collected in accordance with the present invention as compared to collection in a standard EDTA blood collection device. Real Time Reverse Transcriptase PCR technology was used to detect the copy number of RASSF1 A mRNA.
Fig. 6 is a graphic representation showing inhibition of unintended up-regulation of mRNA for glyceraldehydes-3-phosphate dehydrogenase (GADPH) in a blood sample collected in accordance with the present invention as compared to collection in a standard EDTA blood collection device. Molecular beacon for GADPH mRNA was used to detect GADPH mRNA in intact cells using flow cytometry.

### DETAILED DESCRIPTION

In general, the invention herein contemplates a unique approach for the stabilization, isolation, and analysis of cellular RNA. The stabilization step acts to inhibit unwanted gene up-regulations and down-regulations after blood draw and protect the quality of recoverable nucleic acids relative to samples that are not stabilized thereby improving the analytic detection sensitivity of the isolated RNA and their resulting diagnostic capabilities.

In one aspect, the unique approach makes use of a particular composition that includes a preservative optionally in combination with one or more metabolic inhibitors, one or more nuclease inhibitors, one or more metal ion chelators or combinations thereof. In another aspect, the unique approach herein contemplates methods that include a step of contacting a blood sample with the compositions herein. A sample so contacted may thereafter be analyzed. Thus, the method of the present invention may involve the steps stabilizing a blood sample, isolating one or more blood cells from the stabilized blood sample, extracting cellular nucleic acids from the isolated blood cells, and analyzing those cellular nucleic acids for the identification of a disease state. The stabilization step may include contacting the blood sample with a protective agent in an amount and for a time sufficient so that RNA synthesis is inhibited at least partially, if not entirely, for at least two hours. The contact time with the protective agent and amount of protective agent used may also be sufficient so that the blood cells within the blood sample are fixed to substantially prevent contamination of the cellular RNA with cell-free RNA or globin RNA. Further, any cellular RNA that is within the blood cells at the time of the blood draw may be substantially preserved to freeze the mRNA expression pattern of the blood cells substantially as of the time of the blood draw (e.g., no longer than 10 minutes post-blood draw or even no longer than 5 minutes post-blood draw). The isolating process may include lysing the red blood cells and lysing the white blood cells. The isolated white blood cells may then be treated to extract cellular RNA from the isolated white blood cells and the extracted RNA may be analyzed for the presence, absence, or severity of a disease state. The methods disclosed herein allow for the efficient preservation, isolation and analysis of cellular nucleic acids while avoiding contamination with undesirable globin RNA and cell-free nucleic acids that originate at extra-cellular locations in vivo (as compared to cellular RNA that becomes cell-free RNA due to cell metabolism and cell lysis post-blood draw).

The process for improved nucleic acid preservation within a blood sample may employ a step of contacting a blood sample with a protective agent containing one or more preservative agents to maintain the integrity of the components within the sample. Ingredients that may be used as preservative agents include, but are not limited to, diazolidinyl urea, imidazolidinyl urea, dimethoylol-5,5dimethylhydantoin, dimethylol urea, 2-bromo-2.-nitropropane-1,3-diol, oxazolidines, sodium hydroxymethyl glycinate, 5-hydroxymethoxymethyl-1-1aza-3,7-dioxabicyclo[3.3.0]octane, 5-hydroxymethyl-1-laza-3,7dioxabicyclo[3.3.0]octane, 5-hydroxypoly[methyleneoxy]methyl-1-1aza-3, 7dioxabicyclo [3.3.0]octane, quaternary adamantine or any combination thereof. Preferred ingredients are selected from the group consisting of diazolidinyl urea (DU), imidazolidinyl urea (IDU), and any combination thereof. The amount of preservative agent used is generally about 10 to about 400 grams per liter. For example, in certain preferred embodiments the protective agent comprises about 4 to about 10 grams of IDU per 100 ml of buffered salt solution and/or about 1 to about 30 grams of DU per 100 ml of buffered salt solution. The preservative agent may be present in the protective agent in an amount of greater than about 0.01 g per 5ml blood sample post-blood draw. The preservative agent may be present in the protective agent in an amount of less than about 0.20g per 5ml blood sample post-blood draw. The concentration of the preservative agent within the protective agent may be greater than about 5% w/v prior to blood draw. The concentration of the preservative agent within the protective agent may be less than about 40% w/v prior to blood draw.

As used throughout the present teachings, the protective agent composition including the preservative agent discussed above is preferably substantially non-toxic. For example, while many cell preservation techniques make use of formaldehyde products for purposes of fixation, the methods herein (and compositions used herein) are free of separately adding and/or handling of any materially significant concentration (e.g., less than about 1% by weight, more preferably less than about 2000 parts per million, more preferably less than about 1000 parts per million, and still more preferably less than about 500 parts per million) of formaldehyde and/or paraformaldehyde prior to any contact with a blood product sample.

In order to further protect the nucleic acids from degradation, the protective agent may also include one, or any combination of a cell membrane permeablizer, a DNase and/or RNase inhibitor, a metal ion chelator, an oxidative stress neutralizer, a metabolic inhibitor, an amino acid, a cationic polymer or a polyamine. It is also possible that one or more components of the protective agent may be prevented from contacting one or more other components of the protective agent. This may be achieved by adding the one or more components to a blood sample at different times or by adding the one or more components into a container in phases or locations that do not allow the one or more components to contact one another prior to blood draw. This may aid in preventing unwanted reactions between the one or more components prior to contact with a blood sample.

The protective agent may contain a nuclease inhibitor that acts to prevent DNase and/or RNase activity within a blood sample. The nuclease inhibitor is preferably present in an amount sufficient to prevent a decrease in the amount and/or quality of the nucleic acids recoverable from the blood sample as compared with a sample that does not include a nuclease inhibitor. Nuclease inhibitors that may be used include, but are not limited to dithiothreitol (DTT), iodoacetamide, iodoacetic acid, heparin, chitosan, cobalt chloride, diethyl pyrocarbonate, ethanol, aurintricarboxylic acid (ATA), glyceraldehydes, sodium fluoride, ethylenediamine tetraacetic acid (EDTA), formamide, vanadyl-ribonucleoside complexes, macaloid, hydroxylamine-oxygen-cupric ion, bentonite, ammonium sulfate, beta-mercaptoethanol, cysteine, dithioerythritol, tris (2-carboxyethyl) phosphene hydrochloride, a divalent cation such as Mg⁺², Mn⁺², Zn⁺², Fe⁺², Ca⁺², Cu⁺² or any combination thereof. One or more nuclease inhibitors may be present within the protective agent in an amount of more than about 0.1% by weight. A nuclease inhibitor may be present within the protective agent in an amount of less than about 60% by weight. The nuclease inhibitor may be present in the protective agent in an amount of greater than about 0.00008g per 5ml blood sample post-blood draw. The nuclease inhibitor may be present in the protective agent in an amount of less than about 0.2g per 5ml blood sample post-blood draw. The concentration of the nuclease inhibitor within the protective agent may be greater than about 0.016% w/v prior to blood draw. The concentration of the nuclease inhibitor within the protective agent may be less than about 5.0% w/v prior to blood draw. The concentration of the nuclease inhibitor within the protective agent may be greater than about 0.5% w/v prior to blood draw. The concentration of the nuclease inhibitor within the protective agent may be less than about 2.0% w/v prior to blood draw. The concentration of the nuclease inhibitor within the protective agent may be from about 0.2% w/v to about 2.0% w/v prior to blood draw.

The protective agent may also include one or more metabolic inhibitors in a suitable amount to reduce cell metabolism within a blood sample. Metabolic inhibitors that may be used include, but are not limited to glyceraldehyde, dihydroxyacetone phosphate, glyceraldehyde 3-phosphate, 1,3-bisphosphoglycerate, 3-phosphoglycerate, 2- phosphoglycerate, phosphoenolpyruvate, pyruvate and glycerate dihydroxyacetate, sodium fluoride, K₂C₂O₄, or any combination thereof. One or more metabolic inhibitors may be present within the protective agent at a concentration of more than about 0.1% w/v. One or more metabolic inhibitors may be present within the protective agent at a concentration of less than about 40% w/v. One or more metabolic inhibitors may be present within the protective agent at a concentration of more than about 1.0% w/v. A metabolic inhibitor may be present within the protective agent at a concentration of less than about 10% w/v. The concentration of the one or more metabolic inhibitors within the protective agent may be from about 2% w/v to about 6% w/v.

The protective agent may also include one or more chelators capable of bonding with metal ions. The purpose of the one or more chelators is to further minimize nuclease activity and the resulting nucleic acid degradation. RNA cleavage via RNase activity requires the presence of divalent metal ions. The metal ion chelators will act by bonding with the metal ions thereby inactivating the ions and reducing the RNase effect of the metal ions. Possible metal ion chelators for addition to the protective agent include but are not limited to one or any combination of ethylene glycol tetraacetic acid (EGTA), 1,2-bis-(o-Aminophenoxy)-ethane-N,N,-N',N'-tetraacetic acid tetraacetoxy-Methyl ester (BAPTA-AM), dietyldithiocarbamate (DEDTC), ethylenediaminetetraacetic acid (EDTA), dicarboxymethyl- glutamic acid, nitrilotriacetic acid (NTA), or ethylenediaminedisuccinic acid (EDDS). A metal ion chelator may be present within the protective agent at a concentration of more than about 0.1 % w/v. A metal ion chelator may be present within the protective agent at a concentration of less than about 40% w/v. A metal ion chelator may be present within the protective agent at a concentration of more than about 1% w/v. A metal ion chelator may be present within the protective agent at a concentration of less than about 20% by w/v. The concentration of the one or more metal ion chelators may be from about 4% to about 12% w/v.

As mentioned above, it may be possible for the protective agent composition to employ a substance to cause cell membrane permeablization in an effort to increase blood cell uptake of the protective agent thereby improving fixation. A selected permeablization substance should generally function to improve the cell membrane's ability to selectively allow access to the protective agent while maintaining desired cell structure and avoiding damage to cell surface proteins. Examples of such cell permeablization substance may include but are not limited to one or any combination of glycerol, chloroquine, ceteth-15 (C₅₆H₁₁₄O₂₁), Triton X-100 ((C₁₄H₂₂O(C₂H₄O)), or saponin. A permeablization substance may be present within the protective agent in an amount of more than about 0.5% by weight. A permeablization substance may be present within the protective agent in an amount of less than about 40% by weight. A permeablization substance may be present within the protective agent in an amount of greater than about 0.001% by weight. A permeablization substance may be present within the protective agent in an amount of less than about 10% by weight. A permeablization substance may be present within the protective agent in an amount of greater than about 0.3% by weight.

The protective agent may also include a substance that acts to prevent oxidative stress. Nucleic acids and RNA in particular have been found to be highly susceptible to oxidative stress. Thus, the addition of antioxidants and/or reactive oxygen species (ROS) scavengers to the protective agent may help to protect the cellular RNA from the deleterious effects of oxidative damage. As used herein, the term "reactive oxygen species (ROS) scavenger group" refers to a group capable of acting as a scavenger of, or reacting with, superoxide (O₂⁻) or other reactive oxygen species (ROS) including hydroxyl radicals, peroxynitrite, hypochlorous acid and hydrogen peroxide. Additional examples of such antioxidants and reactive oxygen species scavengers include but are not limited to one or any combination of polyphenols such as flavonoids, phenolic acids, D-Mannitol, N-acetyl-L-cysteine, natural phenolic antioxidants (alpha-hydroxytyrosol, tyrosol, caffeic acid, alpha-tocopherol) as well as commercial phenolic antioxidants (BHT and BHA) or carotenoids. An ROS scavenger may be present within the protective agent in an amount of more than about 0.1% by weight. An ROS scavenger may be present within the protective agent in an amount of less than about 40% by weight. An ROS scavenger may be present within the protective agent in an amount of more than about 2% by weight. An ROS scavenger may be present within the protective agent in an amount of less than about 15% by weight.

The protective agent may also include one or more polycations (preferably polyamines) in a suitable amount such that they are capable of binding with any nucleic acids thereby preventing degradation of the nucleic acids. While polycations generally bind to nucleic acids, many polycations also alter cell membrane structure which may be associated with the loss of cell markers located on the cell membrane. Polyamines are naturally synthesized cations that do not compromise the structure of the cell membrane and thus are highly preferred for their ability to bind specifically to cellular RNA, based upon the polyanionic nature of the RNA. In binding to the RNA, the polyamines are able to protect the nucleic acids from RNase activity. The polyamines that may be added include but are not limited to protamine, spermine, spermidine, putrescine, cadaverine, or any combination thereof. The polyamines may be present in the protective agent in an amount of greater than about 0.003g per 5ml blood sample post-blood draw. The polyamines may be present in the protective agent in an amount of less than about 0.1 g per 5ml blood sample post-blood draw. The concentration of the polyamines within the protective agent may be greater than about 77.5 mM prior to blood draw. The concentration of the polyamines within the protective agent may be less than about 1562.5 mM prior to blood draw. The concentration of the polyamines within the protective agent may be greater than about 5% w/v prior to blood draw. The concentration of the polyamines within the protective agent may be less than about 50% w/v prior to blood draw.

Additional classes of cationic compositions (included in the polyamines discussed above) may also be included in the protective agent. Certain cationic polymers are used in DNA transfection processes such as those disclosed in U.S. Patent No. 6,013,240, incorporated by reference herein. The affinity of these cationic polymers in binding with nucleic acids may aid in protecting the nucleic acids from nuclease activity. Cationic polymers that may be used include but are not limited to polylysine, polyamidoamine dendrimer, polyethylenimine, (poly(dimethylamino)ethyl methylacrylate (pDMAEMA), polypropylenimine, or any combination thereof. The PEI may be low molecular weight PEI, such as about 400 g/mol to about 1000 g/mol. The cationic polymers (polyamines) may be present in the protective agent in an amount of greater than about 0.01 g per 5ml blood sample post-blood draw. The polyamines may be present in the protective agent in an amount of less than about 0.1 g per 5ml blood sample post-blood draw. The concentration of the polyamines within the protective agent may be greater than about 5% w/v prior to blood draw. The concentration of the polyamines within the protective agent may be less than about 50% w/v prior to blood draw.

The protective agent may also include one or more amino acids that react in a manner similar to the one or polyamines discussed above. By binding to cellular nucleic acids, the amino acids may protect the nucleic acids from deleterious nuclease activity. The amino acids may include but are not limited to isoleucine, leucine, lysine, valine, tryptophan, threonine, phenylalanine, methionine, alanine, histidine, asparagine, aspartate, cysteine, glutamate, glutamine, glycine, proline, serine, tyrosine, arginine, or any combination thereof. One or more amino acids may be present within the protective agent in an amount of more than about 0.001% by weight. One or more amino acids may be present within the protective agent in an amount of less than about 30% by weight. One or more amino acids may be present within the protective agent in an amount of more than about 0.1% by weight. One or more amino acids may be present within the protective agent in an amount of less than about 10% by weight.

The protective agent may also include one or more protease inhibiting compounds for inhibiting enzyme activity that may have deleterious effects on the integrity of any nucleic acids present in a blood sample. These protease inhibiting compounds may include but are not limited to antipain, aprotinin, chymostatin, elastatinal, phenylmethylsulfonyl fluoride (PMSF), APMSF, TLCK, TPCK, leupeptin, soybean trypsin inhibitor, indoleacetic acid (IAA), E-64, pepstatin, VdLPFFVdL, EDTA, 1,10-phenanthroline, phosphoramodon, amastatin, bestatin, diprotin A, diprotin B, alpha-2-macroglobulin, lima bean trypsin inhibitor, pancreatic protease inhibitor, egg white ovostatin, egg white cystatin or any combination thereof. Combinations of protease inhibitors, commonly referred to as a "protease inhibition cocktail" by commercial suppliers of such inhibitors, may also be used as the stabilizing agent. Such "cocktails" may be generally advantageous in that they provide stabilization for a range of proteins of interest. A protease inhibitor may be present within the protective agent in an amount of more than about 0.1 % by weight. A protease inhibitor may be present within the protective agent in an amount of less than about 40% by weight. A protease inhibitor may be present within the protective agent in an amount of greater than about 0.001% by weight. A protease inhibitor may be present within the protective agent in an amount of less than about 10% by weight. A protease inhibitor may be present within the protective agent in an amount of greater than about 0.1 % by weight.

The protective agent may further include one or more phosphatase inhibitors for inhibiting enzyme activity that may have deleterious effects on the integrity of any nucleic acids present in a blood sample. These phosphatase inhibiting compounds may include but are not limited to calyculin A, nodularin, NIPP-1, microcystin LR, tautomycin, okadaic acid, cantharidin, calyculin A, microcystin LR, okadaic acid, fostriecin, tautomycin, cantharidin, endothall, nodularin, cyclosporin A, FK 506/immunophilin complexes, cypermethrin, deltamethrin, fenvalerate, bpV(phen), dephostatin, mpV(pic) DMHV, sodium orthovanadate or any combination thereof. A phosphatase inhibitor may be present within the protective agent in an amount of more than about 0.1% by weight. A phosphatase inhibitor may be present within the protective agent in an amount of less than about 40% by weight. A phosphatase inhibitor may be present within the protective agent in an amount of more than about 1% by weight. A phosphatase inhibitor may be present within the protective agent in an amount of less than about 20% by weight.

The protective agent or any of the overall compositions may also be substantially free of guanidinium salts, sodium dodecyl sulfate (SDS), or any combination thereof.

The initial contacting of the blood sample will be for a time sufficient to inhibit one or both of cell lysis and nuclease activity, or any combination thereof. Contacting may occur for at least about 10 seconds, more preferably at least about 1 minute, still more preferably at least about 2 minutes. Contacting may also occur for longer periods of time. For example, contacting may be commenced substantially contemporaneously from the time of blood draw (e.g., within less than about 10 minutes of the blood draw) and it may last until nucleic acids are isolated, screened, and/or tested. The contacting step may also be employed to provide a sample with a longer shelf life. Thus, it is possible that a lapse of time of at least about 2 hours, more preferably at least about 6 hours, at least about 24 hours, at least about 7 days or even at least about 14 days can elapse between the time of blood draw (which may be substantially contemporaneous with the contacting step), and the time of any testing or screening of the sample, and or isolation of the nucleic acids. The protective agent may comprise an active agent in solution. Suitable solvents include water, saline, dimethylsulfoxide, alcohol or any mixture thereof. The protective agent may comprise diazolidinyl urea (DU) and/or imidazolidinyl urea (IDU) in a buffered salt solution. The compositions herein (e.g. the protective agent) may further comprise one or more of spermine, spermidine, polyethylenimine, and histidine. The protective agent may contain only a fixative and is free of any additional additives.

The present invention may include one or more preservative agents, one or more metabolic inhibitors, one or more nuclease inhibitors and one or more metal ion chelators. The amount of any preservative agent within the protective agent is generally at least about 10% by weight. The amount of any preservative agent within the protective agent may be generally less than about 70% by weight. The preservative agent may comprise at least about 20% IDU by weight, and generally less than 40% IDU by weight. The preservative agent may comprise at least about 20% DU by weight, and generally less than 40% DU by weight. The protective agent may further contain a metal ion chelator such as at least about 5% EDTA by weight. For example, the protective agent may contain about 8% EDTA by weight. The protective agent may contain less than about 50% EDTA by weight. The protective agent may include from about 0.001% to about 30% by weight of one or more metabolic inhibitors. For example, the protective agent may contain at least about 3% glyceraldehyde by weight and at least about 0.1% sodium fluoride by weight. The protective agent may include from about 0.001% to about 20% by weight of one or more nuclease inhibitors. For example, the protective agent may contain at least about 0.5% aurintricarboxylic acid (ATA) by weight. The protective agent may contain less than about 5% aurintricarboxylic acid (ATA) by weight.

The amount of preservative agent relative to the amount of EDTA is preferably about 1 to about 10 parts (more preferably about 2 to about 5 parts) by weight of preservative agent to about 1 part by weight EDTA. The amount of preservative agent relative to the amount of metabolic inhibitors may be about 1 to about 10 parts (more preferably about 2 to about 8 parts) by weight of preservative agent to about 1 part by weight of metabolic inhibitors. The amount of preservative agent relative to the amount of nuclease inhibitors may be about 1 to about 30 parts (more preferably about 15 to about 22 parts) by weight of preservative agent to about 1 part by weight of nuclease inhibitors. The amount of protective agent within a tube or other receptacle for receiving a biological specimen prior to blood draw is preferably about 300 to 1000 g/liter and more preferably about 400 to about 700 g/ liter.

The combination of one or more preservative agents, one or more metabolic inhibitors, one or more nuclease inhibitors and one or more chelators within the protective agent results in improved ability to maintain the amount and quality of RNA within a blood sample. These results are believed unexpected and superior to results obtained by the use of only the one or more preservative agents, only the one or more metabolic inhibitors, only the one or more nuclease inhibitors, only the one or more chelators or any combination including at least two but less than all of the one or more preservative agents, the one or more metabolic inhibitors, the one or more nuclease inhibitors, or the one or more chelators. Therefore it is contemplated that a synergistic effect occurs when one or more preservative agents, one or more metabolic inhibitors, one or more nuclease inhibitors, and one or more chelators are combined.

Additionally, multiple components of the protective agent may undergo a lyophilization process so that each component is added either prior to or post-blood draw in a substantially solid form to prevent unwanted reactions between one or more components of the protective agent. Similar agents in substantially solid form and associated blood screening devices are disclosed in U.S. Publication No. 2010/0167271 incorporated by reference herein for all purposes. Liquid removal techniques can be performed on the protective agent in order to obtain a substantially solid state protective agent. Liquid removal conditions may preferably be such that they result in removal of at least about 50% by weight, more preferably at least about 75% by weight, and still more preferably at least about 85% by weight of the original amount of the dispensed liquid protective agent. Liquid removal conditions may preferably be such that they result in removal of sufficient liquid so that the resulting composition is in the form of a film, gel or other substantially solid or highly viscous layer; for example it may result in a substantially immobile coating (preferably a coating that can be re-dissolved or otherwise dispersed upon contact with a blood product sample). Thus, liquid removal conditions may preferably be such that they result in a material that upon contact with the sample under consideration (e.g., a blood sample) the protective agent will disperse in the sample, and substantially preserve components (e.g., cellular nucleic acids) in the sample. Liquid removal conditions may preferably be such that they result in a remaining composition that is substantially free of crystallinity; has a viscosity that is sufficiently high that the remaining composition is substantially immobile at ambient temperature (e.g., it does not exhibit any visibly detectable (as seen by the naked eye) flow when held in a storage device at room temperature on an incline of at least about 45° for at least one hour); or both. In this regard as taught in the forgoing application a colorant may also be employed. In one embodiment, one or more polyamines may be combined prior to lyophilization and then lyophilized. One or more preservative agents may also be combined with one or more enzyme inhibitors prior to lyophilization and then the combined preservative agents and enzyme inhibitors may be lyophilized. Lyophilization of one or more polyamines and one or more preservative agents prior to any contact between the one or more polyamines and the one or more preservative agents may prevent any undesired effects (e.g., a loss of cationic function) that may occur during contact in any substantially liquid form.

A blood screening device (e.g., a specimen container) may further include a structure for physically separating any components of the protective agent that should be prevented from contacting one another prior to blood draw. This means may require removal post-blood draw or may simply breakdown upon placement of a blood sample into the specimen container. The blood screening device may include a receptacle that receives a sample of blood and that is substantially transparent over at least a portion of its area. The device may include a first end, a second end, a base portion located a distance between the first end and second end that divides the receptacle into a receiving portion and an elongated channel portion, wherein the first end and second end are both open. The device may further include the protective agent composition placed within the receptacle and being visible through the substantially transparent window, the protective agent composition being in solid form and located in the top portion of the receptacle and being of sufficient concentration so that upon contact with the sample of blood the protective agent composition will disperse in the sample, and substantially preserve white blood cell components in the sample.

The protective agent may be located within a specialized device, wherein the protective agent is already present in the device prior to addition of the blood sample, such as those disclosed in U.S. Patent Publication No. 2004/0137417, incorporated by reference herein. The device may also be an evacuated collection container, such as a tube. The tube may preferably be made of a transparent material that will also resist adherence of the cells within a given sample. The interior wall of the tube may be coated or otherwise treated to modify its surface characteristics, such as to render it more hydrophobic and/or more hydrophilic, over all or a portion of its surface. The tube may have an interior wall flame sprayed, subjected to corona discharge, plasma treated, coated or otherwise treated. The tube may be treated by contacting an interior wall with a substance so that the nucleic acids of interest will resist adhering to the tube walls. The surface of the tube may be modified to provide a dual functionality that simultaneously provides an appropriate balance of desired hydrophilicity and hydrophobicity, to allow collection of blood, dispersion of the preservatives herein, and resistance of adhesion of nucleic acids to the inner wall of a blood collection tube. Thus it is possible that any coating may be a functionalized polymeric coating that includes a first polymer and one or more second monomeric and/or polymeric functionalities that are different from (e.g., chemically different from) the first polymer. The coating may include one or more co-polymers (e.g., block copolymer, graft copolymer, or otherwise). For example, it may include a copolymer that includes a first hydrophobic polymeric portion, and a second hydrophilic polymeric portion. The coating may be a water based coating. The coating may optionally include an adhesion promoter. The coating may be applied in any suitable manner, it may be sprayed, dipped, swabbed, or otherwise applied onto some or all of the interior of the blood collection tube. The coating may also be applied in the presence of heat. Preferably any coating applied to the inner wall of a blood collection tube will form a sufficiently tenacious bond with the glass (e.g., borosilicate glass) or other material (e.g., polymeric material) of the tube so that it will not erode or otherwise get removed from the inner wall. Examples of suitable polymeric coatings may include silicon containing polymers (e.g., silanes, siloxanes, or otherwise); polyolefins such as polyethylene or polypropylene; polyethylene terephthalate; fluorinated polymers (e.g., polytetrafluoroethylene); polyvinyl chloride, polystyrene or any combination thereof. Examples of teachings that may be employed to coat an interior of a blood collection tube may be found in U.S. Patent Nos. 6,551,267; 6,077,235; 5,257,633; and 5,213,765; all incorporated by reference.

The protective agent may also be placed prior to or post-blood draw into a receptacle employing a blood sample identification system such as that disclosed in co-owned U.S. Application Serial No. 12/871,955 entitled "Blood Sample Identification System" incorporated by reference herein for all purposes. The identification system includes a handling device for a biological specimen within a receptacle comprising an initially planar substrate including at least one crease that divides the substrate into a handle portion having at least one peripheral edge portion and a receiving portion that includes at least a portion of an aperture having a perimeter that is configured so that it receives a container having a cover that includes a biological specimen for test, and resists pull-through of the container relative to the substrate. The substrate may further include identification information about the sample contained within the receptacle and/or its source.

As discussed above, the tube may include a metal ion chelator (which may also be an anticoagulant agent), one or more metabolic and/or nuclease inhibitors and a preservative agent such as a fixative agent including but not limited to those disclosed above. The tube may also include one or any combination of one or more polyamines, a cell membrane permeablizer, and an antioxidant or reactive oxygen species scavenger. Preferably, the compounds included in the tube are in an amount sufficient to preserve cell morphology and prevent cell degradation while also preventing deleterious DNase and RNase activity within the nucleic acids. In preferred embodiments, blood may be fixed simultaneously as it is drawn into the specialized tube. The tube may also be coated over an exterior wall with a protective coating (e.g., a containment barrier that helps control glass shard fragmentation) such as that disclosed in U.S Patent No. 7,419,832, incorporated by reference herein.

As discussed herein, a step of contacting a blood sample with the protective agent allows the sample to be stored for a period of time prior to isolating and testing the nucleic acids. A blood sample may be drawn at one location, contacted with the protective agent, and later transported to a different remote or off-site location for the nucleic acid isolation and testing process. The nucleic acids may be isolated from the blood sample and tested at the remote location and the resulting diagnostic information is then reported to the site of the original blood draw. The nucleic acid isolation process may be performed at one remote location and the resulting data can be analyzed to identify the presence, absence or relative severity of a disease state at a third location. Alternatively the results of the nucleic acid isolation process may be sent back to the site of the initial blood draw and analyzed there. The resulting diagnostic information may then be sent to a third location or back to the remote location or the site of the initial blood draw. The blood draw location and the testing site and/or analysis site are separated by at least about 0.5 km, 1km, 100km, or longer.

At any time after the initial contact of the blood sample with the protective agent, the sample can be treated to isolate one or more blood cells from the sample and extract the cellular nucleic acids located within the isolated blood cells. The nucleic acids may be extracted and isolated using any method including those methods disclosed in commonly owned US Publication No. 2009/0081678 incorporated by reference herein. The preservative agent acts to prevent cell lysis so that the blood cells remain intact and substantially all cellular nucleic acids remain intra-cellular to avoid unwanted contamination with cell-free RNA and globin RNA.

After the cellular RNA has been extracted, it can be tested to identify the presence, absence or severity of a disease state. The methods herein thus further contemplate a step of nucleic acid testing. Testing of the nucleic acids can be performed using any nucleic acid testing method including, but not limited to polymerase chain reaction (PCR), reverse transcription polymerase chain reaction (RT-PCR), quantitative real time polymerase chain reaction (Q-PCR), gel electrophoresis, capillary electrophoresis, mass spectrometry, fluorescence detection, ultraviolet spectrometry, DNA hybridization, allele specific polymerase chain reaction, polymerase cycling assembly (PCA), asymmetric polymerase chain reaction, linear after the exponential polymerase chain reaction (LATE-PCR), helicase-dependent amplification (HDA), hot-start polymerase chain reaction, intersequence-specific polymerase chain reaction (ISSR), inverse polymerase chain reaction, ligation mediated polymerase chain reaction, methylation specific polymerase chain reaction (MSP), multiplex polymerase chain reaction, nested polymerase chain reaction, solid phase polymerase chain reaction, or any combination thereof.

The present invention also contemplates a method for isolating and testing cellular RNA. The method can be performed on a single sample or on a multitude of samples (e.g., in a multi-well plate). The method includes contacting a blood sample with a protective agent. The protective agent includes a preservative agent as previously discussed so that the blood cells remain intact throughout the blood draw and RNA isolation process. The protective agent further includes a component to protect the RNA from RNase activity or to inhibit RNase activity altogether. Post-blood draw, the blood sample may be contacted with a red blood cell lysis buffer which may include NH₄Cl, KHCO_{3.} and EDTA in water. The blood sample may then be placed in ice water for about 15 to about 20 minutes. The sample may then be centrifuged at about 1000 rpm at about 1°C to about 10°C for about 10 minutes. The supernatant may then be removed and a red blood cell lysis buffer may be added for removal of red blood cells. The resulting white blood cell pellet may then be resuspended and centrifuged at about 1000 rpm at about 1°C to about 10°C for about 10 minutes. The supernatant may then be removed by aspiration in an effort to avoid any disruption to the white blood cell pellet. The RNA may then be isolated using any nucleic acid isolation method. As an example, the AllPrep DNA/RNA Mini Kit manufactured by QIAGEN, Inc. of Valencia, California may be used. The white blood cell pellet may be initially contacted with a cell lysis buffer which may contain quanidine hydrochloride and β-mercaptoethanol. The cell pellet may then be vortexed to promote cell lysis. After cell lysis, cell lysate is introduced to a homogenizing device by microcentrifuge at about 13000 rpm at room temperature for about 1 minute to about 4 minutes to ensure disruption and homogenization of the cell lysate. The homogenized cell lysate may then be applied to a DNA binding column and microcentrifuged at about 8000 g at room temperature for about 1 minute to remove the majority of DNA. The flow-through may then be collected and mixed with ethanol to adjust the salt concentration and pH for proper binding on the RNA column. The mixture may then be applied to an RNA column and contacted with one or more buffers to remove any impurities including protein and salts. The RNA may then be eluted by RNase-free water and stored at about -80°C for long term storage or at about 0°C for short term storage. For use in a UV spectrophotometer, the RNA samples may be kept at 0°C but analyzed at room temperature. For use in a bioanalyzer, the RNA samples may first be denatured at about 70°C for about 2 minutes then immediately cooled at about 0°C on ice to keep the RNA denatured and free of any tertiary structure. The RNA samples may remain on ice until loaded onto the bioanalyzer chips for analysis at room temperature.

### Example 1

Blood samples from the same donor are drawn into two separate blood collection tubes (tube 1 (EDTA tube) and tube 2 (RNA BCT tube)). Tube 1 contains only EDTA. Tube 2 contains DU, EDTA, ATA, glyceraldehyde and sodium fluoride. Both tubes are stored at room temperature and 1ml aliquots of blood are removed from each tube at hours 1.5, 8, 24, 48, 72 and 96. The blood glucose levels of each sample are measured using a YSI blood glucose meter available from YSI Life Sciences (Yellow Springs, OH). The blood glucose concentration of samples from tube 2 were the only samples that maintained relatively consistent glucose levels over the test period, indicating that the combination of EDTA, DU, ATA, glyceraldehyde and sodium fluoride provided reduced levels of cell metabolism. The results of this example are shown in a graphic format at Figure 1.

### Example 2

Blood samples from the same donor are drawn into two separate blood collection tubes (tube 1 and tube 2). Tube 1 contains EDTA. Tube 2 contains DU, EDTA, ATA, glyceraldehyde and sodium fluoride. Both tubes are stored for 2 h at room temperature before plasma was separated. RNase activity of plasma from tube 1 and tube 2 was measured using a commercially available RNase activity detection kit, RNaseAlert®Lab Test Kit (Applied Biosystems, Foster City, CA). Two additional control experiments were also carried out with purified RNase A enzyme alone and RNase A treated with chemical mixture present in tube 2. RNase activity is presented as relative fluorescence. Results of this example are illustrated in a graphic format at Figure 2.

### Example 3

Two blood samples from the same donor are drawn into two separate blood collection tubes, tube A (RNA BCT) and tube B (EDTA). Tube A contains DU, EDTA, ATA, glyceraldehyde and sodium fluoride. Tube B contains only EDTA. Both tubes are stored at room temperature and 5ml aliquots of blood are removed from each tube on day 0, day 1, day 2, and day 3 and plasma is separated. All samples are centrifuged at 800 g for 10 minutes at room temperature to separate the plasma. The plasma is then transferred into new tubes and centrifuged at 1500 g for 10 minutes at room temperature. Free circulating RNA is purified using the QIAamp circulating nucleic acid kit available from Qiagen Inc. (Valencia, CA). RNA is extracted from each plasma sample. The samples are then amplified by Real Time PCR (using TaqMan® RT PCR reagents available from Applied Biosystems, Foster City, California) to identify the c-fos mRNA copy number per ml of plasma. Results showed a consistent copy number of c-fos mRNA per ml of plasma in samples originating from tube A at each measurement, indicating little or no change in c-fos mRNA level in tube A over a 3 day period. The c-fos mRNA copy number per ml of plasma showed elevated levels at every measurement in those samples originating in tube B, indicating an increase in the amount of c-fos mRNA present as a result of increased cell metabolism. The results of this example are shown in a graphic format at Figure 3.

### Example 4

Two blood samples from the same donor are drawn into two separate blood collection tubes, tube A (RNA BCT) and tube B (EDTA). Tube A contains DU, EDTA, ATA, glyceraldehyde and sodium fluoride. Tube B contains only EDTA. Both tubes are stored at room temperature and 5ml aliquots of blood are removed from each tube on day 0, day 1, day 2, and day 3 and plasma is separated. All samples are centrifuged at 800 g for 10 minutes at room temperature to separate the plasma. The plasma is then transferred into new tubes and centrifuged at 1500 g for 10 minutes at room temperature. Free circulating RNA is purified using the QIAamp circulating nucleic acid kit available from Qiagen Inc. (Valencia, CA). RNA is extracted from each plasma sample. The samples are then amplified by Real Time PCR (using TaqMan® RT PCR reagents available from Applied Biosystems, Foster City, California) to identify the GAPDH mRNA copy number per ml of plasma. Results showed a consistent copy number of GAPDH mRNA per ml of plasma in samples originating in tube A at each measurement, indicating little or no change in GAPDH mRNA level in tube A over a 3 day period. The GAPDH mRNA copy number per ml of plasma showed elevated levels at every measurement in those samples originating in tubes B, indicating an increase in the amount of GAPDH mRNA present as a result of increased cell metabolism. The results of this example are shown in a graphic format at Figure 4.

### Example 5

Two blood samples from the same donor are drawn into two separate blood collection tubes, tube A (RNA BCT) and tube B (EDTA). Tube A contains DU, EDTA, ATA, glyceraldehyde and sodium fluoride. Tube B contains only EDTA. Both tubes are stored at room temperature and 5ml aliquots of blood are removed from each tube on day 0, day 1, day 2, and day 3 and plasma is separated. All samples are centrifuged at 800 g for 10 minutes at room temperature to separate the plasma. The plasma is then transferred into new tubes and centrifuged at 1500 g for 10 minutes at room temperature. Free circulating RNA is purified using the QIAamp circulating nucleic acid kit available from Qiagen Inc. (Valencia, CA). RNA is extracted from each plasma sample. The samples are then amplified by Real Time PCR (using TaqMan® RT PCR reagents available from Applied Biosystems, Foster City, California) to identify the RASSF1A mRNA copy number per ml of plasma. Results showed a consistent copy number of RASSF1A mRNA per ml of plasma at each measurement, indicating little or no change in c-fos mRNA level in tube A over a 3 day period. The RASSF1A mRNA copy number per ml of plasma showed declined at every measurement in those samples originating in tubes B, indicating an decrease in the amount of RASSF1A mRNA present as a result of RASSF1A mRNA down-regulation. The results of this example are shown in a graphic format at Figure 5.

### Example 6

Two blood samples from the same donor are drawn into two separate blood collection tubes, tube A (RNA BCT) and tube B (EDTA). Tube A contains DU, EDTA, ATA, glyceraldehyde and sodium fluoride. Tube B contains only EDTA. Both tubes are stored at room temperature and 5ml aliquots of blood are removed from each tube on day 0, day 1, day 2, and day 3 and white blood cells were Isolated by either density gradient centrifugation or by removing red cells using a red cell lysis solution. White blood cells isolated from tube A and tube B were treated with ice-cold 100% methanol for 10 min separately. Then cells from both tubes were washed with PBS for two times and suspended in PBS and incubated with a molecular beacon for GAPDH mRNA for 1 h at room temperature. After this incubation period cells from both tube A and tube B were analyzed by flowcytometry to quantify the GADPH mRNA Level in intact white blood cells. Results showed a consistent level of GAPDH mRNA indicating little or no change in GAPDH mRNA level in tube A over a 3 day period. The GAPDH mRNA level showed elevated at every measurement in those samples originating in tubes B, indicating an increase in the amount of GAPDH mRNA present as a result of increased cell metabolism. The results of this example are shown in a graphic format at Figure 6.

Examples 1 through 6 above demonstrate an unexpected synergistic effect occurring in blood samples contacted by a preservative, one or more metabolic inhibitors and/or nuclease inhibitors, and one or more chelators. Blood samples contacted by only a preservative, only one or more metabolic inhibitors and/or nuclease inhibitors, only one or more chelators, or any combination of only some but not all of those do not demonstrate the ability to maintain the integrity of the blood cells or the integrity of the nucleic acids. The combined effect of the DU and one or more metabolic and/or nuclease inhibitors far exceeds any expectations based on the effect, or lack thereof, of the DU or one or more metabolic and/or nuclease inhibitors used alone.

Any numerical values recited herein include all values from the lower value to the upper value in increments of one unit provided that there is a separation of at least 2 units between any lower value and any higher value. As an example, if it is stated that the amount of a component or a value of a process variable such as, for example, temperature, pressure, time and the like is, for example, from 1 to 90, preferably from 20 to 80, more preferably from 30 to 70, it is intended that values such as 15 to 85, 22 to 68, 43 to 51, 30 to 32 etc. are expressly enumerated in this specification. For values which are less than one, one unit is considered to be 0.0001, 0.001, 0.01 or 0.1 as appropriate. These are only examples of what is specifically intended and all possible combinations of numerical values between the lowest value and the highest value enumerated are to be considered to be expressly stated in this application in a similar manner. As can be seen, the teaching of amounts expressed as "parts by weight" herein also contemplates the same ranges expressed in terms of percent by weight. Thus, an expression in the Detailed Description of the Invention of a range in terms of at "'x' parts by weight of the resulting polymeric blend composition" also contemplates a teaching of ranges of same recited amount of "x" in percent by weight of the resulting polymeric blend composition."

Unless otherwise stated, all ranges include both endpoints and all numbers between the endpoints. The use of "about" or "approximately" in connection with a range applies to both ends of the range. Thus, "about 20 to 30" is intended to cover "about 20 to about 30", inclusive of at least the specified endpoints.

The disclosures of all articles and references, including patent applications and publications, are incorporated by reference for all purposes. The term "consisting essentially of" to describe a combination shall include the elements, ingredients, components or steps identified, and such other elements ingredients, components or steps that do not materially affect the basic and novel characteristics of the combination. The use of the terms "comprising" or "including" to describe combinations of elements, ingredients, components or steps herein also contemplates embodiments that consist essentially of the elements, ingredients, components or steps. By use of the term "may" herein, it is intended that any described attributes that "may" be included are optional.

Plural elements, ingredients, components or steps can be provided by a single integrated element, ingredient, component or step. Alternatively, a single integrated element, ingredient, component or step might be divided into separate plural elements, ingredients, components or steps. The disclosure of "a" or "one" to describe an element, ingredient, component or step is not intended to foreclose additional elements, ingredients, components or steps. All references herein to elements or metals belonging to a certain Group refer to the Periodic Table of the Elements published and copyrighted by CRC Press, Inc., 1989. Any reference to the Group or Groups shall be to the Group or Groups as reflected in this Periodic Table of the Elements using the IUPAC system for numbering groups.

It will be appreciated that concentrates or dilutions of the amounts recited herein may be employed. In general, the relative proportions of the ingredients recited will remain the same. Thus, by way of example, if the teachings call for 30 parts by weight of a Component A, and 10 parts by weight of a Component B, the skilled artisan will recognize that such teachings also constitute a teaching of the use of Component A and Component B in a relative ratio of 3:1. Teachings of concentrations in the examples may be varied within about 25% (or higher) of the stated values and similar results are expected. Moreover, such compositions of the examples may be employed successfully in the present methods.

It will be appreciated that the above is by way of illustration only. Other ingredients may be employed in any of the compositions disclosed herein, as desired, to achieve the desired resulting characteristics. Examples of other ingredients that may be employed include antibiotics, anesthetics, antihistamines, preservatives, surfactants, antioxidants, unconjugated bile acids, mold inhibitors, nucleic acids, pH adjusters, osmolarity adjusters, or any combination thereof.

It is understood that the above description is intended to be illustrative and not restrictive. Many embodiments as well as many applications besides the examples provided will be apparent to those of skill in the art upon reading the above description. The scope of the invention should, therefore, be determined not with reference to the above description, but should instead be determined with reference to the appended claims, along with the full scope of equivalents to which such claims are entitled. The disclosures of all articles and references, including patent applications and publications, are incorporated by reference for all purposes. The omission in the following claims of any aspect of subject matter that is disclosed herein is not a disclaimer of such subject matter, nor should it be regarded that the inventors did not consider such subject matter to be part of the disclosed inventive subject matter.

## Claims

1. A screening method for the identification of a disease state, comprising the steps of:
a. contacting a drawn blood sample that includes a plurality of blood cells with an RNA protective agent in an amount and for a time sufficient so that:
i. RNA synthesis is inhibited for at least about two hours;
ii.blood cells of the drawn blood sample are fixed to substantially prevent contamination of cellular RNA with cell-free RNA or globin RNA;
iii. any cellular RNA that is within the blood cells at the time of the blood draw is substantially preserved to freeze the protein expression pattern of the blood cells substantially as of the time of the blood draw;
b. isolating white blood cells from the drawn blood sample; and
c. extracting cellular RNA from the isolated white blood cells.

2. The method of claim 1, wherein the protective agent includes a preservative agent selected from the group consisting of: diazolidinyl urea, imidazolidinyl urea, dimethoylol-5,5dimethylhydantoin, dimethylol urea, 2-bromo-2.-nitropropane-1,3-diol, oxazolidines, sodium hydroxymethyl glycinate, 5-hydroxymethoxymethyl-1-1aza-3,7-dioxabicyclo [3.3.0]octane, 5-hydroxymethyl-1-1 aza-3,7dioxabicyclo[3.3.0]octane, 5-hydroxypoly[methyleneoxy]methyl-1-1 aza-3, 7dioxabicyclo[3.3.0]octane, quaternary adamantine and any combination thereof.

3. The method of claim 1 or claim 2, wherein the protective agent includes imidazolidinyl urea.

4. The method of any of claims 1 through 3, wherein the protective agent includes diazolidinyl urea.

5. The method of any of claims 1 through 4, wherein the protective agent includes one or more metabolic inhibitors selected from the group consisting of: dihydroxyacetone phosphate, glyceraldehyde 3-phosphate, 1,3-bisphosphoglycerate, 3-phosphoglycerate, 2- phosphoglycerate, phosphoenolpyruvate, pyruvate and glycerate dihydroxyacetate, sodium fluoride, K₂C₂O₄ and any combination thereof.

6. The method of any of claims 1 through 5, wherein the protective agent includes glyceraldehyde and sodium fluoride.

7. The method of any of claims 1 through 6, wherein the protective agent includes one or more nuclease inhibitors selected from the group consisting of: dithiothreitol (DTT), iodoacetamide, iodoacetic acid, heparin, chitosan, cobalt chloride, diethyl pyrocarbonate, ethanol, aurintricarboxylic acid (ATA), glyceraldehydes, sodium fluoride, ethylenediamine tetraacetic acid (EDTA), formamide, vanadyl-ribonucleoside complexes, macaloid, hydroxylamine-oxygen-cupric ion, bentonite, ammonium sulfate, beta-mercaptoethanol, cysteine, dithioerythritol, tris (2-carboxyethyl) phosphene hydrochloride, a divalent cation such as Mg⁺², Mn⁺², Zn⁺², Fe⁺², Ca⁺², Cu⁺², and any combination thereof.

8. The method of any of claims 1 through 7, wherein the protective agent includes aurintricarboxylic acid.

9. The method of any of claims 1 through 8, wherein the protective agent includes one or more metal ion chelators selected from the group consisting of: ethylene glycol tetraacetic acid (EGTA), 1,2-bis-(o-Aminophenoxy)-ethane-N,N,-N',N'-tetraacetic acid tetraacetoxy-Methyl ester (BAPTA-AM), dietyldithiocarbamate (DEDTC), ethylenediaminetetraacetic acid (EDTA), dicarboxymethyl- glutamic acid, nitrilotriacetic acid (NTA), ethylenediaminedisuccinic acid (EDDS), and any combination thereof.

10. The method of any of claims 1 through 9, wherein the protective agent includes EDTA.

11. The method of any of claims 2 though 10, wherein the concentration of the preservative agent prior to the contacting step is about 1 % w/v to about 65% w/v.

12. The method of any of claims 2 through 11, wherein the preservative agent is diazolidinyl urea and has a concentration of about 15% w/v to about 35% w/v prior to the contacting step.

13. The method of any of claims 5 through 12, wherein the concentration of the one or more metabolic inhibitors prior to the contacting step is about 0.1% w/v to about 15% w/v.

14. The method of any of claims 7 though 13, wherein the concentration of the one or more nuclease inhibitors prior to the contacting step is about 0.1 % w/v to about 15% w/v.

15. The method of any of claims 9 through 14, wherein the concentration of the one or more metal ion chelators prior to the contacting step is about 1 % w/v to about 25% w/v.

16. The method of any of claims 1 through 15, wherein (i) either or both of the isolating or analyzing steps occurs at least 1 day after the blood sample is drawn, (ii) either or both of the isolating or analyzing steps occurs without freezing the blood sample (e.g. to a temperature colder than about -30°C (more preferably colder than about -70°C)); or both (i) and (ii).

17. A screening method for the identification of a disease state, comprising the steps of:
contacting a drawn blood sample that includes a plurality of blood cells with an RNA protective agent comprising:
i. one or more preservative agents;
ii. one or more nuclease inhibitors;
iii. one or more metabolic inhibitors;
iv. one or more metal ion chelators;
isolating white blood cells from the drawn blood sample; and
extracting cellular RNA from the isolated white blood cells.

18. The method of claim 17, wherein the preservative agent is diazolidinyl urea and has a concentration of about 15% w/v to about 35% w/v prior to the contacting step.

19. The method of claim 17 or claim 18, wherein the concentration of the one or more metabolic inhibitors prior to the contacting step is about 0.1% w/v to about 15% w/v.

20. The method of any of claims 17 through 19, wherein the concentration of the one or more nuclease inhibitors prior to the contacting step is about 0.1% w/v to about 15% w/v.

21. The method of any of claims 17 through 20, wherein the concentration of the one or more metal ion chelators prior to the contacting step is about 1% w/v to about 25% w/v.

22. The method of any of claims 17 though 21, wherein (i) either or both of the isolating or analyzing steps occurs at least 1 day after the blood sample is drawn, (ii) either or both of the isolating or analyzing steps occurs without freezing the blood sample (e.g. to a temperature colder than about -30°C (more preferably colder than about -70°C)); or both (i) and (ii).

23. A device for receiving a blood sample, the device being preloaded with a protective agent comprising:
i. one or more preservative agents having a concentration of about 15% w/v to about 35% w/v;
ii. one or more nuclease inhibitors having a concentration of about 0.1 % w/v to about 15% w/v;
iii. one or more metabolic inhibitors having a concentration of about 0.1 % w/v to about 15% w/v;
iv. one or more metal ion chelators having a concentration of about 1 % w/v to about 25% w/v.
